(19) Europäisches Patentamt
European Patent Office
Office européen des brevets

(11) EP 0 850 043 B1

(12) EUROPEAN PATENT SPECIFICATION

(45) Date of publication and mention of the grant of the patent:
**30.01.2002 Bulletin 2002/05**

(21) Application number: **96928199.7**

(22) Date of filing: **16.08.1996**

(51) Int Cl.[7]: **A61K 7/48**

(86) International application number:
**PCT/US96/13263**

(87) International publication number:
**WO 97/07779 (06.03.1997 Gazette 1997/11)**

(54) **SKIN CONDITIONING COMPOSITION CONTAINING SILICONE GUM, MIXTURE OF POLYOXYETHYLENES AND GLYCEROL COMPOUND**

HAUTPFLEGEMITTEL. DAS SILIKONGUMMI SOWIE EINE MISCHUNG AUS POLYOXIETHYLENEN UND EINER GLYCERINVERBINDUNG ENTHALT

COMPOSITION DE CONDITIONNEMENT DE LA PEAU CONTENANT UNE GOMME DE SILICONE UN MELANGE DE POLYOXYETHYLENES ET UN COMPOSE DE GLYCEROL

(84) Designated Contracting States:
**AT BE CH DE DK ES FI FR GB GR IE IT LI LU NL PT SE**

(30) Priority: **31.08.1995 US 522481**

(43) Date of publication of application:
**01.07.1998 Bulletin 1998/27**

(73) Proprietor: **THE PROCTER & GAMBLE COMPANY
Cincinnati, Ohio 45202 (US)**

(72) Inventors:
- **GLASGO, Timothy, Jay
Williamsburg, OH 45176 (US)**
- **RANA, Fazale, Rahman
West Chester, OH 45069 (US)**
- **JEVTITCH, Milan, Marcel
Cincinnati, OH 45208 (US)**
- **DODD, Michael, Thomas
Edgewood, KY 41018 (US)**

(74) Representative: **McKinney, Victoria et al
Patent Dept., Procter & Gamble Technical Centres Limited, Rusham Park, Whitehall Lane
Egham TW20 9NW (GB)**

(56) References cited:
**EP-A- 0 613 682**
**WO-A-92/09263**
**GB-A- 2 275 419**

- **CHEMICAL ABSTRACTS, vol. 120, no. 8, 21 February 1994 Columbus, Ohio, US; abstract no. 86122, M.AIZAWA: "Skin cosmetics containing silicone polymers and polyethylene glycol" XP002022321 & JP,A,05 262 618 (SHISEIDO)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

## Description

### FIELD OF INVENTION

This invention relates to topical skin care compositions. More particularly, this invention relates to topical aqueous skin care compositions containing a combination of polyoxyethylenes, a glycerol or polyglycerol, and silicone.

### BACKGROUND OF THE INVENTION

A wide variety of skin care compositions are topically applied for conditioning and moisturizing the skin. Exemplary types of such compositions include hand and facial conditioning compositions, sunscreen compositions, after-shave compositions, and skin renewal compositions.

It is desirable for these products to provide a variety of different effects, including those from a functional standpoint and from an aesthetics (or skin feel) standpoint. From a functional standpoint, it is desirable for the products to condition the skin, so that it feel smooth to the touch. Emollients are commonly used in skin care products for this purpose. Emollients coat the skin, sealing in moisture and providing a modified skin feel after application. It is also desirable for the products to moisturize the skin. Humectants are commonly used in skin care products to moisturize the skin. In addition to reducing dry skin feel and dry/flaky skin appearance, humectants also help reduce roughness due to dry skin, which can result in increased incidence of cuts due to shaving. Ideally, skin care products provide immediate benefits of moisturization and conditioning, as well as long term moisturization and conditioning.

In addition to the functional benefits of moisturization and conditioning, it is important for skin care products to have a pleasing skin feel. In particular, it should not feel sticky or tacky when applied to or when on the skin. Unfortunately, many ingredients which are highly effective for moisturizing or conditioning the skin, e.g. emollients such as nonvolatile silicones and humectants such as glycerol, tend to feel sticky or tacky during application to the skin, or subsequently, after being applied to the skin. Although eliminating silicone emollients and humectants can reduce stickiness and tackiness, that also reduces product performance.

In addition to the above concerns, silicone emollients are generally insoluble in aqueous skin care compositions, and consequently require suspension aids so that they remain stably dispersed in the products. Unfortunately, many thickening agents used in such products can also degrade skin feel of the products. The most commonly used are crosslinked acrylic acid/acrylate polymers such as carbomers, or other hydrophilic polymers which tend to feel cold and wet on the skin.

It is an object of this invention to provide skin care compositions which have improved combinations functional properties, including conditioning and moisturizing, along with excellent skin feel and low stickiness and tackiness.

It is another object of this invention to provide skin care compositions, as described immediately above, which are thickened with hydrophilic polymers yet still exhibit low stickiness and tackiness.

It is yet another object of this invention to provide skin care compositions, as described immediately above, which have low stickiness and tackiness, and are easily distributed across the surface of the skin.

It is yet another object of this invention to provide skin care compositions in accordance with the above objects which provide an immediate sense of skin conditioning and moisturizing upon application and a long term sense of conditioning and moisturizing.

It is yet another object of this invention to provide skin care compositions as provided immediately above, which have improved long term moisturization.

It is another object of this invention to provide a product as described above which further exhibits excellent skin sensitivity.

These and other benefits as may be discussed herein or apparent to one skilled in the art can be provided according to the invention which is described below.

All percentages herein are by weight of the composition unless otherwise indicated. All ratios are weight ratios unless otherwise indicated. All percentages, ratios, and levels of ingredients referred to herein are based on the actual amount of the ingredient, and do not include solvents, fillers, or other materials with which the ingredient may be combined as commercially available products, unless otherwise indicated. All average molecular weight values are number averages, unless otherwise indicated.

The invention hereof can comprise, consist of, or consist essentially of the essential elements described herein as well as any of the preferred or optional ingredients also described herein. Nothing herein is meant to be interpreted to exclude the use or presence of other components or steps except as may be otherwise indicated.

### SUMMARY OF THE INVENTION

The present invention provides a thickened, aqueous composition suitable for topical application to the skin.

The present compositions comprise a unique combination of water, a hydrogel forming polymeric gelling agent, water-insoluble high molecular weight silicone emollient, a glycerol component, a low molecular weight polyethylene glycol, and a high molecular weight polyoxyethylene. The compositions can provide short term and extended term skin conditioning and moisturization in combination with excellent skin feel and low tackiness.

More specifically, the present invention provides a topical skin conditioning composition comprising:

(a) from about 0.1% to about 10%, by weight, hydrogel forming polymeric gelling agent, calculated on dry polymeric gelling agent weight basis, said gelling agent not having a Tg below 140°C;
(b) an insoluble silicone emollient dispersed in said composition, said silicone emollient comprising a mixture of (i) from about 0.05% to about 2%, by weight of the composition, of a water insoluble silicone gum; and (ii) from about 0.5% to about 15%, by weight of the composition, of a water insoluble diluent having a viscosity at 25°C of about 400 mPa·s (centipoise) or less;
(c) from about 0.5% to 10%, by weight, glycerol, a polymer of glycerol having a weight average molecular weight of about 800 or less, alkyl ether derivatives thereof, or a combination thereof;
(d) from about 0.5% to about 30%, by weight, of polyethylene glycol having a number average degree of ethoxylation of from about 2 to about 12;
(e) from about 0.05% to about 2%, by weight, polyoxyethylene having the structure:

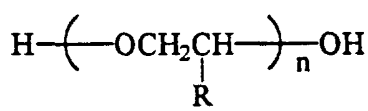

wherein n has a number average of from about 800 to about 20,000, and R is hydrogen or $C_1$ to $C_4$ alkyl, or a combination thereof; and
(f) from about 50% to about 98.7%, by weight, water.

The present invention, as well as various optional and preferred embodiments thereof, are described in more detail below.

DETAILED DESCRIPTION OF THE INVENTION

Hydrogel Forming Polymeric Gelling Agent

The compositions will comprise an aqueous gel, i.e. a "hydrogel", formed from a hydrogel forming polymeric gelling agent and water. The compositions will contain from about 0.1% to about 10%, by weight of the composition, preferably from about 0.2% to about 5%, more preferably from about 0.3% to about 1%, of such hydrogel forming polymeric gelling agent, calculated based on the dry weight of the hydrogel forming polymeric gelling agent.

In general, the hydrogel forming polymeric gelling agent materials of the present invention are at least partially crosslinked polymers prepared from polymerizable, unsaturated acid-containing monomers which are water-soluble or become water-soluble upon hydrolysis. These include monoethylenically unsaturated compounds having at least one hydrophilic radical, including (but not limited to) olefinically unsaturated acids and anhydrides which contain at least one carbon-carbon olefinic double bond. With respect to these monomers, water-soluble means that the monomer is soluble in deionized water at 25°C at a level of at least 0.2%, preferably at least 1.0%.

Upon polymerization, monomeric units as described above will generally constitute from about 25 mole percent to 99.99 mole percent, more preferably from about 50 mole percent to 99.99 mole percent, most preferably at least about 75 mole percent of the polymeric gelling agent material (dry polymer weight basis), of acid-containing monomers.

The hydrogel forming polymeric gelling agent herein is partially crosslinked to a sufficient degree preferably that is high enough such that the resulting polymer does not exhibit a glass transition temperature (Tg) below about 140°C, and accordingly, the term "hydrogel forming polymeric gelling agent," as used herein, shall mean polymers meeting this parameter. Preferably the hydrogel forming polymeric gelling agent does not have a Tg below about 180°C, and more preferably does not have a Tg prior to decomposition of the polymer, at temperatures of about 300°C or higher. The Tg can be determined by differential scanning calorimetry (DSC) conducted at a heating rate of 20.0 C°/minute with 5 mg or smaller samples. The Tg is calculated as the midpoint between the onset and endset of heat flow change corresponding to the glass transition on the DSC heat capacity heating curve. The use of DSC to determine

Tg is well known in the art, and is described by B. Cassel and M. P. DiVito in "Use of DSC To Obtain Accurate Thermodynamic and Kinetic Data", American Laboratory, January 1994, pp 14-19, and by B. Wunderlich in Thermal Analysis, Academic Press, Inc., 1990.

The hydrogel forming polymeric material hereof is also characterized by being highly absorbent and able to retain water in its absorbed or "gel" state. Preferred hydrogel forming polymeric gelling agent hereof will be able to absorb at least about 40 g water (deionized) per gram of gelling agent, preferably at least about 60 g/g, more preferably at least about 80 g/g. These values, referred to as "Absorptive Capacity" herein can be determined according to the procedure in the Absorptive Capacity "Tea Bag" test in the Experimental Section below.

The hydrogel forming polymeric gelling agent hereof will also preferably be characterized by an extractable polymer content of no more than about 20%, more preferably no more than about 12%, most preferably no more than about 10%. The extractable polymer content is determined according to the procedures set forth in the Experimental Section, below.

Examples of acid-containing water-soluble monomers from which monomer units of the polymers hereof can be derived from include carboxylic acid- and sulfonic acid-containing monomers, such as:

1. Carboxyl group-containing monomers (carboxylic acid-containing): monoethylenically unsaturated mono or poly-carboxylic acids, such as (meth) acrylic acid (meaning acrylic acid or methacrylic acid; similar notations are used hereinafter), maleic acid, fumaric acid, sorbic acid, itaconic acid, citraconic acid, tricarboxy ethylene, and ethacrylic acid;
2. Carboxylic acid anhydride group-containing monomers: monoethylenically unsaturated polycarboxylic acid anhydrides, such as maleic anhydride;
3. Carboxylic acid salt-containing monomers: water-soluble salts (alkali metal salts, ammonium salts, amine salts, etc.) of monoethylenically unsaturated mono- or poly- carboxylic acids [such as sodium (meth)acrylate, trimethylamine (meth)acrylate, triethanolamine (meth) acrylate, sodium maleate, methylamine maleate];
4. Sulfonic acid group-containing monomers: aliphatic or aromatic vinyl sulfonic acids (such as vinylsulfonic acid, allyl sulfonic acid, vinyltoluenesulfonic acid, styrene sulfonic acid), (meth)acrylic sulfonic acids [such as sulfopropyl (meth) acrylate, 2-hydroxy-3-(meth)acryloxy propyl sulfonic acid, 2-acylamido-2-methyl propane sulfonic acid];
5. Sulfonic acid salt group-containing monomers: alkali metal salts, ammonium salts, amine salts of sulfonic acid group-containing monomers as mentioned above.

Preferred monomers include carboxylic acid monomers, or anhydrides or salts thereof. Especially preferred monomers include acrylic acid, methacrylic acid, and maleic acid, and anhydrides and salts thereof. Acrylic acid and combinations of acrylic acid and acrylate salt (e.g. sodium acrylate) are particularly preferred.

Two or more different monomer types of the previously described acid group-containing monomers may be copolymerized in order to provide the hydrogel-forming polymeric gelling material.

Monomers which become water-soluble by hydrolysis, for use in this invention include monethylenically unsaturated compounds having at least one hydrolyzable group, such as ester and nitrile groups. Such monomers having an ester group include for example, lower alkyl (C1-C3) esters of monoethylenically unsaturated carboxylic acids, such as methyl (meth)acrylate, ethyl (meth)acrylate and 2-ethylhexyl (meth)acrylate; and esters of monoethylenically unsaturated alcohols [vinyl esters, (meth)-allyl ester, etc.], such as vinyl acetate and (meth) allyl acetate. Suitable nitrile group-containing monomers include (meth) acrylonitrile.

While at least 25 mole percent (preferably at least about 35%, more preferably at least about 50%) of the hydrogel-forming polymer compositions herein should be prepared from acid group-containing monomers, some non-acid monomers may also be used to prepare the hydrogel-forming polymer compositions herein (prior to neutralization). Such non-acid monomers can include, for example, the water-soluble or water-dispersible esters of the foregoing acid-containing monomers as well as monomers which contain no carboxyl or sulfonic acid groups at all. Optional non-acid monomers can thus include, for example, carboxylic acid or sulfonic acid ester-containing monomers e.g. $C_1$-$C_4$ esters, hydroxyl group-containing monomers, amide group-containing monomers, amino group-containing monomers, nitrile group containing monomers, quaternary ammonium salt group-containing monomers and unsaturated polymerizable hydrocarbons, such as alph-olefins, vinyl monomers, and vinylidene monomers. Examples include ethylene, propylene, isobutylene, 1-butylene, vinyl acetate, methyl vinyl ether, isobutyl vinyl ether, and styrene compounds of the formula:

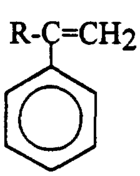

wherein R represents H or a $C_1$-$C_6$ alkyl, and wherein the benzene ring may be substituted with low molecular weight alkyls (e.g. $C_1$-$C_4$) or hydroxy groups.

Exemplary types of such water soluble or hydrophilic moiety-containing monomers as set forth above that can be used include: hydrophilic groups of such monomers.

1. Hydroxyl group-containing monomers: monoethylenically unsaturated alcohols [such as (meth)allyl alcohol], monoethylenically unsaturated ethers or esters of polyols (alkylene glycols, glycerol, polyoxyalkylene polyols), such as hydroxethyl (meth)acrylate, hydroxypropyl (meth)acrylate, triethylene glycol (meth)acrylate, poly(oxyethylene oxypropylene) glycol mono (meth)allyl ether (in which hydroxyl groups may be etherified or esterified).
2. Amide group-containing monomers: (meth) acrylamide, N-alkyl (meth)acrylamides (such as N-methylacrylamide, N-hexylacrylamide), N,N-dialkyl (meth)acryl amides (such as N,N-dimethylacrylamide, N,N'-di-n-propylacrylamide), N-hydroxyalkyl (meth)acrylamides [such as N-methylol(meth)acrylamide, N-hydroxyethyl (meth)acrylamide], N,N-dihydroxyalkyl (meth)acrylamides [such as N,N-dihydroxyethyl (meth)acrylamide], vinyl lactams (such as N-vinylpyrrolidone);
3. Amino group-containing monomers: amino group-containing esters (e.g. dialkylaminoalkyl esters, dihydroxyalkylaminoalkyl esters, morpholinoalkyl esters, etc.) of monoethylenically unsaturated mono- or dicarboxylic acid [such as dimethlaminoethyl (meth)acrylate, diethylaminoethyl (meth)acrylate, morpholinoethyl (meth)acrylate, dimethyl aminoethyl fumarate], heterocyclic vinyl compounds [such as vinyl pyridines (e.g. 2-vinyl pyridine, 4-vinyl pyridine, N-vinyl pyridine), N-vinyl imidazol]; and
4. Quaternary ammonium salt group-containing monomers: N,N,N-trialkyl-N-(meth)acryloyloxyalkylammonium salts [such as N,N,N-trimethyl-N-(meth)acryloyloxyethylammonium chloride, N,N,N-triethyl-N-(meth)acryloyloxyethylammonium chloride, 2-hydroxy-3-(meth)-acryloyloxypropyl trimethyl ammonium chloride], and monomers as mentioned in British patent specification No. 1,034,296.

These non-acid monomers are well known materials and are further described in, for example, Masuda et al., U.S. Patent 4,076,663, issued February 28, 1978; and in Westerman, U.S. Patent 4,062,817, issued December 13, 1977; and U.S. Patent 5,151,465 Le-Khac, issued September 29, 1992. If present, such non-acid monomers will generally be used only to such an extent that, prior to neutralization, no more than 75% mole percent of the polymer compositions herein are prepared from such non-acid monomers, preferably no more than about 65%, more preferably no more than about 50%.

In the hydrogel-forming polymeric gelling agent, the polymeric component formed from unsaturated, acid-containing monomers may be grafted on to other types of polymer moieties such as starch or cellulose.

Suitable starches include, for example, natural starches such as sweet potato starch, potato starch, wheat starch, corn starch, rice starch, tapioca starch, and the like, and processed or modified starches such as alpha-starch, dextrine, oxidized starch, dialdehyde starch, alkyl-etherified starch, allyl-etherified starch, oxyalkylated starch, aminoethyl-etherified starch, cyanoethyl-etherified starch and the like.

Suitable celluloses include, for example, celluloses obtained from wood, leaves, stems, bast, seed fluffs, and the like; and modified celluloses such as alkyl-etherified cellulose, organic-acid-esterified cellulose, oxidized cellulose, hydrocellulose, and the like. Starch grafted materials of this type are especially preferred for use herein.

The hydrogel forming polymeric gelling agent hereof will, in general, be at least partially crosslinked. Suitable cross-linking agents are well know in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer material; and (4) polyvalent metal compounds which can form ionic cross-linkages.

Cross-linking agents having at least two polymerizable double bonds include (i) di- or polyvinyl compounds such as divinylbenzene and divinyltoluene; (ii) di- or poly-esters of unsaturated mono- or poly-carboxylic acids with polyols including, for example, di- or triacrylic acid esters of polyols such as ethylene glycol, trimethylol propane, glycerine, or polyoxyethylene glycols; (iii) bisacrylamides such as N,N-methylenebisacrylamide; (iv) carbamyl esters that can be obtained by reacting polyisocyanates with hydroxyl group-containing monomers; (v) dior poly-allyl ethers of polyols; (vi) di- or poly-allyl esters of polycarboxylic acids such as diallyl phthalate, diallyl adipate, and the like; (vii) esters of unsaturated mono- or poly-carboxylic acids with mono-allyl esters of polyols such as acrylic acid ester of polyethylene glycol monoallyl ether; and (viii) di- or tri-allyl amine.

Cross-linking agents having at least one polymerizable double bond and at least one functional group reactive with the acid-containing monomer material include N-methylol acrylamide, glycidyl acrylate, and the like. Suitable cross-linking agents having at least two functional groups reactive with the acid-containing monomer material include glyoxal; polyols such as ethylene glycol and glycerol; polyamines such as alkylene diamines (e.g., ethylene diamine), polyalkylene polyamines, polyepoxides, di- or polyglycidyl ethers and the like. Suitable polyvalent metal cross-linking agents which can form ionic cross-linkages include oxides, hydroxides and weak acid salts (e.g., carbonate, acetate and the

like) of alkaline earth metals (e.g., calcium, magnesium) and zinc, including, for example, calcium oxide and zinc diacetate.

Cross-linking agents of many of the foregoing types are described in greater detail in Masuda et al., U.S. Patent 4,076,663, issued February 28, 1978, and Allen et al., U.S. Patent 4,861,539, issued August 29. Preferred cross-linking agents include the di- or polyesters of unsaturated mono- or polycarboxylic acids mono-allyl esters of polyols, the bisacrylamides, and the di- or tri-allyl amines. Specific examples of especially preferred cross-linking agents include N,N'-methylenebisacrylamide and trimethylol propane triacrylate.

The cross-linking agent will generally constitute from about 0.001 mole percent to 5 mole percent of the resulting hydrogel-forming polymeric material. More generally, the cross-linking agent will constitute from about 0.01 mole percent to 3 mole percent of the hydrogel-forming polymeric gelling agent used herein.

The hydrogel forming polymeric gelling hereof may be employed in their partially neutralized form. For purposes of this invention, such materials are considered partially neutralized when at least 25 mole percent, and preferably at least 50 mole percent of monomers used to form the polymer are acid group-containing monomers which have been neutralized with a base. Suitable neutralizing bases cations include hydroxides of alkali and alkaline earth metal (e.g. KOH, NaOH), ammonium, substituted ammonium, and amines such as amino alcohols (e.g., 2-amino-2-methyl-1,3-propanediol, diethanolamine, and 2-amino-2-methyl-1-propanol. This percentage of the total monomers utilized which are neutralized acid group-containing monomers is referred to herein as the "degree of neutralization." The degree of neutralization will preferably not exceed 98%.

Hydrogel forming polymeric gelling agents suitable for use herein are well known in the art, and are described, for example, in U.S. Patent 4,076,663, Masuda et al., issued February 28, 1978; U.S. Patent 4,062,817, Westerman, issued December 13, 1977; U.S. Patent 4,286,082, Tsubakimoto et al., issued August 25, 1981; U.S. Patent 5,061,259, Goldman et al., issued October 29, 1991, and U.S. Patent 4,654,039, Brandt et al., issued March 31, 1987.

Hydrogel forming polymeric gelling agents suitable for use herein are also described in U.S. Patent 4,731,067, Le-Khac, issued March 15, 1988, U.S. Patent 4,743,244, Le-Khac, issued May 10, 1988, U.S. Patent 4,813,945, Le-Khac, issued March 21, 1989, U.S. Patent 4,880,868, Le-Khac, issued November 14, 1989, U.S. Patent 4,892,533, Le-Khac, issued January 9, 1990, U.S. Patent 5,026,784, Le-Khac, issued June 25, 1991, U.S. Patent 5,079,306, Le-Khac, issued January 7, 1992, U.S. Patent 5,151,465, Le-Khac, issued September 29, 1992, U.S. Patent 4,861,539, Allen, Farrer, and Flesher, issued August 29, 1989, and U.S. Patent 4,962,172, Allen, Farrer, and Flesher, issued October 9, 1990.

Suitable hydrogel forming polymeric gelling agents in the form of particles are commercially available from Hoechst Celanese Corporation, Portsmouth, VA, USA (Sanwet™ Superabsorbent Polymers) Nippon Shokubai, Japan (Aqualic™, e.g., L-75, L-76) and Dow Chemical Company, Midland, MI, USA (Dry Tech™).

Hydrogel forming polymeric gelling agents in the form of fibers are commercially available from Camelot Technologies Inc., Leominster, MA, USA (Fibersorb™, e.g., SA 7200H, SA 7200M, SA 7000L, SA 7000, and SA 7300).

The compositions hereof may also contain other hydrophilic gelling agents. These include carboxylic acid-containing polymers as otherwise described above, except which have relatively lower degrees of crosslinking, such that they exhibit a Tg below 140°C, as well as a variety of other water soluble or colloidally water soluble polymers, such as cellulose ethers (e.g. hydroxyethyl cellulose, methyl cellulose, hydroxy propylmethyl cellulose), polyvinylpyrrolidone, polyvinylalcohol, guar gum, hydroxypropyl guar gum and xanthan gum. Preferred among these additional hydrophilic gelling agents are the acid-containing polymers, particularly carboxylic acid-containing polymers. Especially preferred are those that comprise water-soluble polymer of acrylic acid crosslinked with a polyalkenyl polyether of a polyhydric alcohol, and optionally an acrylate ester or a polyfunctional vinylidene monomer.

Preferred copolymers useful in the present invention are polymers of a monomeric mixture containing 95 to 99 weight percent of an olefinically unsaturated carboxylic monomer selected from the group consisting of acrylic, methacrylic and ethacrylic acids; about 1 to about 3.5 weight percent of an acrylate ester of the formula:

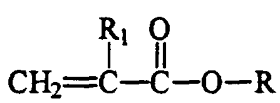

wherein R is an alkyl radical containing 10 to 30 carbon atoms and $R_1$ is hydrogen, methyl or ethyl; and 0.1 to 0.6 weight percent of a polymerizable cross-linking polyalkenyl polyether of a polyhydric alcohol containing more than one alkenyl ether group per molecule wherein the parent polyhydric alcohol contains at least 3 carbon atoms and at least 3 hydroxyl groups.

Preferably, these polymers contain from about 96 to about 97.9 weight percent of acrylic acid and from about 2.5 to about 3.5 weight percent of acrylic esters wherein the alkyl group contains 12 to 22 carbon atoms, and $R_1$ is

methyl, most preferably the acrylate ester is stearyl methacrylate. Preferably, the amount of crosslinking polyalkenyl polyether monomer is from about 0.2 to 0.4 weight percent. The preferred crosslinking polyalkenyl polyether monomers are allyl pentaerythritol, trimethylolpropane diallylether or allyl sucrose. These polymers are fully described in U.S. Patent No. 4,509,949, to Huang et al., issued April 5, 1985.

Other preferred copolymers useful in the present invention are the polymers which contain at least two monomeric ingredients, one being a monomeric olefinically-unsaturated carboxylic acid, and the other being a polyalkenyl, polyether of a polyhydric alcohol. Additional monomeric materials may be present in the monomeric mixture if desired, even in predominant proportion.

The first monomeric ingredient useful in the production of these carboxylic polymers are the olefinically-unsaturated carboxylic acids containing at least one activated carbon-to-carbon olefinic double bond, and at least one carboxyl group. The preferred carboxylic monomers are the acrylic acids having the general structure

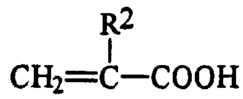

wherein $R^2$ is a substituent selected from the class consisting of hydrogen, halogen, and the cyanogen (-C=N) groups, monovalent alkyl radicals, monovalent alkaryl radicals and monovalent cycloaliphatic radicals. Of this class, acrylic, methacrylic, and ethacrylic acid are most preferred. Another useful carboxylic monomer is maleic anhydride or the acid. The amount of acid used will be from about 95.5 to about 98.9 weight percent.

The second monomeric ingredient useful in the production of these carboxylic polymers are the polyalkenyl polyethers having more than one alkenyl ether grouping per molecule, such as alkenyl groups in which an olefinic double bond is present attached to a terminal methylene grouping, $CH_2$=C<.

The additional monomeric materials which may be present in the polymers include polyfunctional vinylidene monomers containing at least two terminal $CH_2$< groups, including for example, butadiene, isoprene, divinyl benzene, divinyl naphthlene, allyl acrylates, and the like. These polymers are fully described in U.S. Patent No. 2,798,053, to Brown, H.P., issued July 2, 1957, this patent being incorporated herein by reference.

Examples of carboxylic acid copolymers useful in the present invention include Carbomer 934, Carbomer 941, Carbomer 950, Carbomer 951, Carbomer 954, Carbomer 980, Carbomer 981, Carbomer 1342, acrylates/C10-30 alkyl acrylate cross polymer (available as Carbopol 934, Carbopol 941, Carbopol 950, Carbopol 951, Carbopol 954, Carbopol 980, Carbopol 981, Carbopol 1342, and the Pemulen series, respectively, from B. F. Goodrich).

Other carboxylic acid copolymers useful in the present invention include sodium salts of acrylic acid/acrylamide copolymers sold by the Hoechst Celanese Corporation under the trademark of Hostaceren PN73. Also included are the hydrogel polymers sold by Lipo Chemicals Inc. under the trademark of HYPAN hydrogels. These hydrogels consist of crystalline plicks of nitrates on a C-C backbone with various other pendant groups such as carboxyls, amides, and amidines. An example would include HYPAN SA 100 H, a polymer powder available from Lipo Chemical.

Neutralizing agents for use in neutralizing the acidic groups of these polymers include those previously described.

The hydrophilic gelling agents characterized by the absence of a Tg below 140°C are highly preferred in the present invention in that they have extremely low tackiness, and therefore cooperate with the other essential ingredients of the invention to provide a highly effective moisturizing composition that combines both short and long term conditioning and moisturizing with low tackiness. More conventional gelling agents, such as those having Tg's below 140°C, on the other hand, provide excellent spreading and ease of application of the composition to the skin, but tend to impart a tacky feel, when used with the skin conditioning ingredients of the present invention. Therefore, in view of the unexpected ability of the present conditioning components to provide both short and long term conditioning without wet, tacky skin feel, it is particularly critical for the present invention to use thickening agents which suspend the insoluble conditioning ingredients but which also do not feel wet and tacky. The hydrophilic gelling agents hereof not having Tg below 140°C are therefore critical to the present invention.

In a preferred embodiment, a mixture of hydrophilic gelling agents comprising the hydrogel forming polymer hereof characterized by having no Tg below 140°C and a second, acid-containing hydrogel forming polymer characterized by having a Tg below 140°C are used in combination, at a weight ratio of the first polymer:second polymer of from 100:0 to 50:50, preferably from about 90:10 to 60:40, more preferably from about 75:25 to 65:35. Such combinations can provide low tackiness as well as improved spreading and ease of application, compared to the use of the first polymer alone.

Water

The compositions will comprise from about 50% to about 98.75%, by weight, water, preferably from about 65% to about 98%, more preferably from about 80% to about 95%. Water is an essential component in the present invention for forming the hydrogel with the hydrogel forming polymeric gelling agent described above.

Silicone Emollient

The compositions hereof will contain a silicone emollient which comprises: (i) from about 0.05% to about 2%, by weight of the composition, of a water insoluble silicone gum; and (ii) from about 0.5% to about 15%, by weight of the composition, of a water insoluble diluent having a viscosity of about 400 centipoise or less, at 25°C; wherein the mixture of (i) and (ii) is dispersed in the composition in the form of droplets. By water insoluble, what is meant is that the material forms a separate phase in water such as in the form of droplets. The compositions will preferably comprise from about 0.1% to about 1.5% of (i), more preferably from about 0.25% to about 1%, and from about 1% to about 10% of (ii), more preferably from about 2% to about 5%. The silicone emolliens preferably has a viscosity of from about 100 mPa·s (centipoise) to about 50,000 mPa·s (centipoise), more preferably from about 2000 to about 8000 mPa·s (centipoise), most preferably from about 3000 to about 6000 mPa·s (centipoise) at 25°C. The viscosity can be measured by the ASTM D 445 standard method, or equivalent. Preferably, the weight ratio of (i):(ii) is from about 5:95 to about 40:60, more preferably from about 10:90 to about 17:83, most preferably from about 10:90 to about 15:85.

The silicone gum hereof is a nonvolatile, water insoluble silicone fluid having a weight average molecular weight of at least about 250,000. The upper limit is not critical except for practical concerns relating to availability and processability of the material In general, the weight average molecular weight of the silicone gum will be no greater than about 1,000,000, preferably from about 275,000 to about 800,000. Weight average moleculare weight of the silicone gum can be determined by gel permeation chromatography, or equivalent.

The number average particle size of the droplets containing the silicone emollient should preferably be from about 1 micron to about 4,000 microns. In order to enhance translucent appearance, it is preferred that the average particle size be at least about 10 micron, preferably at least about 25 microns, more preferably at least about 40 microns. It is also preferable for the average droplet size be about 1000 microns or less, more preferably about 500 microns or less, most preferably about 250 microns or less. Particle size can be measured by a light scattering particle size analyzer or equivalent.

The silicone gums suitable for use herein include polyalkylsiloxanes and polyalkyaryl siloxanes, including linear and branched chained silicones.

The silicone gums suitable for purposes hereof includes those represented by general formula (I):

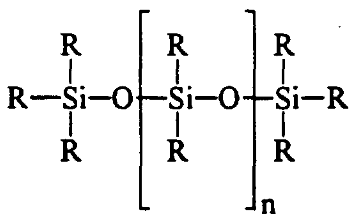

wherein each R independently is substituted or unsubstituted aliphatic (e.g. alkyl or alkenyl), aryl, aryloxy, alkaryl, alkamino (e.g. alkyl or alkenyl amino groups), hydroxy, or hydrogen, or combinations thereof; and n is an integer, preferably from about 3000 to about 10,000, more preferably from about 4,000 to about 8,000, most preferably from about 5000 to about 7000. The R substituents can also include combinations of hydroxy groups and amine groups, as well as other functional groups, such as halogens (preferably fluorine) and halogen-substituted functionalities, e.g. halogen-substituted aliphatic and aryl groups. The structures can also include alkoxy functionalities, as long as the silicone remains insoluble in water.

Preferred alkyl and alkenyl substitutes are $C_1$-$C_5$ alkyls and alkenyls, more preferably from $C_1$-$C_4$, most preferably from $C_1$-$C_2$. The aliphatic portions of other alkyl-, alkenyl-, or alkynyl- containing groups (such as alkaryl, and alkamino) can be straight or branched chains and preferably have from one to five carbon atoms, more preferably from one to four carbon atoms, even more preferably from one to three carbon atoms, most preferably from one to two carbon atoms. As discussed above, the R substituents hereof can also, contain amino functionalities, e.g., alkamino groups, which can be primary, secondary or tertiary amines or quaternary ammonium. These include mono-, di-, and tri- alkylamino and alkoxyamino groups wherein the aliphatic portion chain length is preferably as described above. The R substituents can also be substituted with other groups, such as halogens (e.g. chloride, fluoride, and bromide),

halogenated aliphatic or aryl groups, and hydroxy (e.g. hydroxy substituted aliphatic groups). Suitable halogenated R groups could include, for example, tri-halogenated (preferably fluoro) alkyl groups such as $-R^1-C(F)_3$, wherein $R^1$ is $C_1$-$C_3$ alkyl. Examples of such polysiloxanes include polymethyl -3,3,3 trifluoropropylsiloxane.

Aryl-containing substituents contain alicyclic and heterocyclic five and six membered aryl rings, and substituents containing fused five or six membered rings. The aryl rings themselves can be substituted or unsubstituted. Substituents include aliphatic substituents, and can also include alkoxy substituents, acyl substituents, ketones, halogens (e.g., Cl and Br), amines, etc. Exemplary aryl-containing groups include substituted and unsubstituted arenes, such as phenyl, and phenyl derivatives such as phenyls with $C_1$-$C_5$ alkyl or alkenyl substituents, e.g., allylphenyl, methyl phenyl and ethyl phenyl, vinyl phenyls such as styrenyl, and phenyl alkynes (e.g. phenyl $C_2$-$C_4$ alkynes). Heterocyclic aryl groups include substituents derived from furan, imidazole, pyrrole, pyridine, etc. Fused aryl ring substituents include, for example, napthalene, coumarin, and purine.

Especially preferred is dimethiconol, of the following structure:

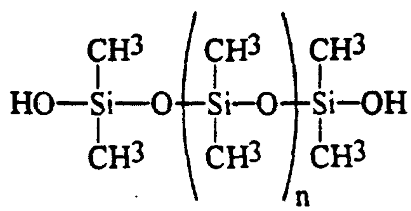

where n is from about 3000 to about 10,000, preferably at least about 4000.

The dispersed droplets comprising the silicone gum also comprise a viscosity-reducing diluent.

Diluents for the silicone gum are water insoluble fluids having a viscosity of about 400 centipoise or less, at 25°C, including those in which the silicone gum is miscible. The diluent can be either volatile or nonvolatile. For purposes hereof, nonvolatile shall mean materials with a vapor pressure at 25°C of less than 0.2 mm Hg. Volatile, as used herein, means materials having a vapor pressure at 25°C of 0.2 mm HG or higher. Preferably the diluent will have a viscosity of from about 0.65 centipoise to about 100 centipoise at 25°C, more preferably from about 0.65 centipoise to about 50 centipoise, most preferably from about 0.65 centipoise to about 15 centipoise. viscosity is determined according to ASTM D standard method, or equivalent, as previously discussed. The diluents hereof include silicone fluids and hydrocarbon fluids. The silicone diluents hereof include linear (branched or straight) chain silicone compounds, including those having the general structure "I" above, where n is zero or an integer from 1 to about 150, where R is as defined above, preferably $C_1$-$C_3$ alkyl, preferably methyl.

Cyclic polysiloxanes include those represented by the general formula:

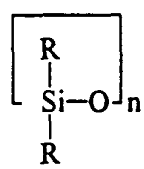

wherein R is as defined above, preferably methyl, n is an integer from 3 to 7, preferably from 3 to 5. The substituents on the siloxane chain (R) can be as defined above and preferably is methyl.

Suitable hydrocarbon fluids include $C_{7-20}$ branched chain hydrocarbons, preferably $C_{10}$-$C_{18}$, more preferably $C_{12}$-$C_{16}$. Examples include isododecane, isohexadecane, isoparaffins, and isolicosane.

<u>Low Molecular Weight Polyethylene Glycol</u>

The compositions of the present invention will comprise from about 0.5% to about 30%, by weight, of one or more low molecular weight polyethylene glycol ("PEG") preferably from about 1% to about 30%, more preferably from about 5% to about 10%.

The low molecular weight are characterized by the general formula (II):

$$H-\left(-OCH_2CH_2-\right)_n-OH$$

wherein: n is an integer, wherein the number average of n is from about 2 to about 12. In this formula, n relates to the degree of ethoxylation. Preferred is where n has a number average of from about 3 to about 10, more preferably from about 4 to about 8, most preferably about 4. The number average degree of ethoxylation (number average n) can be determined by supercritical fluid chromatography (SFC), gas chromatography (GC), or equivalent.

High Molecular Weight Polyoxyethylene

The compositions of the present invention will comprise from about 0.05% to about 2%, by weight, of one or more high molecular weight polyoxyethylenes (hereafter "POE's"), preferably from about 0.05% to about 1%, more preferably from about 0.10% to about 0.35%, most preferably from about 0.15% to about 0.25%.

The high molecular weight polyoxyethylene compounds used herein have a structure according to the following formula (III):

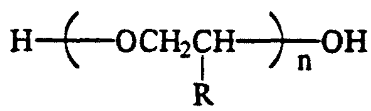

wherein: R is hydrogen or $C_1$ to $C_4$ alkyl preferably hydrogen or methyl, more preferably hydrogen, or a mixture thereof; and n is an integer of from 800 to about 20,000. In this formula, n relates to the degree of ethoxylation. Preferred is where n has a number average of from about 1000 to about 10,000, more preferably from about 1500 to about 5000, as determined by gel permeation chromatography, or equivalent.

When R is hydrogen, the compound is polyethylene glycol ( (PEG). Exemplary polyethylene glycol polymers exemplary are PEG-2M wherein R equals H and n has an average value of about 2,000 (PEG 2-M is also known as Polyox WSR® N-10 from Union Carbide and as PEG-2,000); PEG-5M wherein R equals H and n has an average value of about 5,000 (PEG 5-M is also known as Polyox WSR® N-35 and Polyox WSR® N-80, both from Union Carbide and as PEG-5,000 and Polyethylene Glycol 300,000); PEG-7M wherein R equals H and n has an average value of about 7,000 (PEG 7-M is also known as Polyox WSR® N-750 from Union Carbide); PEG-9M wherein R equals H and n has an average value of about 9,000 (PEG 9-M is also known as Polyox WSR® N-3333 from Union Carbide); and PEG-14 M wherein R equals H and n has an average value of about 14,000 (PEG 14-M is also known as Polyox WSR® N-3000 and N-205 from Union Carbide.)

Other useful polymers include the polypropylene glycols and mixed polyethylene/polypropylene glycols.

Glycerol Component

The compositions of the present invention will comprise from about 0.5% to about 10%, by weight, of a glycerol component, preferably from about 1% to about 7%, more preferably from about 1.5% to about 4%.

The glycerol component hereof includes: glycerol (i.e., $HOCH_2CH(OH)CH_2OH$); polymers of glycerol having a weight average molecular weight of about 800 or less; derivatives thereof, and mixtures thereof. Preferred weight average molecular weight for polyglycerol is about 500 or less, more preferably about 300 or less.

Derivatives of glycerol and polyglycerol are alkyl ether derivatives (preferably $C_1$-$C_{18}$, more preferably $C_1$-$C_{16}$, most preferably $C_1$-$C_4$). Exemplary alkyl ether derivatives include ethyl, propyl, and butyl ether derivatives. Exemplary propylether derivatives are disclosed in U.S. Patent No. 4,976,953, issued December 11, 1990 to Orr and Sabatelli, wherein the propoxylated ether derivatives of glycerol contain one or two degrees of propoxylation.

Mole Ratios of Glycerol Component:Low Molecular Weight Polyethylene Glycol

The mole ratio of the glycerol component to the low molecular weight polyethylene glycol should preferably be from about 1:2 to about 2:1, more preferably from about 2:3 to about 3:2, even more preferably from about 3:4 to about 4:3, most preferably about 1:1. These mole ratios are based upon the number average molecular weight of any polymeric compounds utilized in the glycerol component and polyethylene glycol. These ratios are preferred for providing unexpected enhanced extended term skin moisturization for the compositions of the present invention.

Optional Ingredients

The present compositions can comprise a wide variety of optional ingredients. Such optional ingredients can be used, for example, for aesthetic purposes, cosmetic purposes, medicinal purposes, skin-protection purposes, or processing ease. Exemplary optional materials are described below. When used, optional ingredients should be present at levels sufficient to provide the intended benefit. In general they will be added at a level of about 0.05%, or higher depending upon the efficacy of the particular ingredient.

Perfume

The compositions of the present invention will optionally comprise a perfume. The perfume will generally be used at a level of from about 0.01% to about 5%, by weight of the composition, preferably from about 0.05% to about 3%, more preferably from about 0.1% to about 1%. Water soluble and water insoluble perfumes can be used. In a particularly preferred embodiment hereof, water insoluble perfume is combined in the water insoluble silicone emollient droplet phase of the present invention, whereby it is suspended in the composition along with the emollient.
By water insoluble perfume, what is meant is that the perfume forms a distinct phase in deionized water at 25°C in the absence of a surfactant or other solubilizer. Water insoluble perfumes will, in general, either separate to form a distinct phase or form a turbid solution in deionized water, at 25°C, at a concentration of 1.0%, by weight, perfume.
Water soluble perfumes, on the other hand, are those perfumes which are not water insoluble as defined herein. By perfume, what is meant is a volatile, odoriferous, component of one or more active perfume compounds which exudes a pleasant or otherwise desired aroma at ambient conditions, or which mask odors.
The perfume active compounds are typically incorporated into the perfume components in liquid form, but can also be solids (such as the various camphoraceous perfumes known in the art) which are solubilized in other ingredients of the perfume component.
In addition to the perfume active compounds, the perfume hereof can also include additional ingredients such as diluents, solvents for solid perfume ingredients, and fixatives, etc. Diluents may or may not have their own aroma and, to the extent that they do, they are categorized as perfume active compounds. Exemplary diluents and solvents include alcohols (e.g. ethyl alcohol, benzyl alcohol, dipropylene glycol, etc.) and liquid hydrocarbon and hydrocarbon esters (e.g., benzyl benzoate and other hydrocarbons and esters described above).

Additional Conditioning Agents

Additional conditioning agent can also be included in the present compositions. Optional conditioning agents suitable for use include both humectants and non-silicone emollients. In general, such additional conditioning agents are used at levels of up to about 20%, by weight of the composition, preferably up to about 10%. When added to provide functional benefits, they will generally be added at a level of at least about 0.05%, preferably at least about 0.1 %.
Exemplary conditioning agents include $C_3$-$C_6$ diols and triols, e.g., propylene glycol, hexylene glycol, 1,3-dihydroxypropane, butylene glycol, 1,4-dihydroxyhexane, 1,2,6-hexane triol, and the like.
Other conditioning agents include: other polyhydroxy alcohols, such as sorbitol; sugars and starches, and derivatives thereof, such as alkoxylated sugars and starches, e.g., alkoxylated glucose; aloe vera; panthenol; hyaluronic acid; lactamide monoethanolamine; octamide monoethanolamine; polypropylene glycol polymers (PPG) and copolymers of PEG and PPG (e.g., PEG/PPG 17/6 copolymer).
Water-insoluble emollient conditioning agents, including hydrocarbons, fatty esters and alcohols, and the like can also be used in addition to the silicone emollient. The average particle size of dispersed phases containing them should preferably be within the ranges provided above.

Sensates and Coolants

A wide variety of coolants and sensate materials can be used. Sensates include menthol, acyclic carboxamides such as those described in U.S. Patent 4,230,688, Rowsell et al., issued October 28, 1980, incorporated herein by reference, e.g., N,2,3-trimethyl-2-isopropylbutanamide, and N-substituted-p-menthane-3-carboxamides such as those described in U.S. Patent 4,136,163, Watson et al., issued January 23, 1979, incorporated herein by reference, e.g., N-ethyl-p-menthane-3-carboxamide. Sensates are generally used at levels of from about 0.01%, to about 2%, by weight, preferably from 0.05% to about 1.0%, more preferably from about 0.05% to about 0.5%. Coolants include volatile liquids that evaporate on the skin, thereby resulting with a cooling sensation. Preferred coolants include ethanol, isopropyl alcohol, and witch hazel. The level of such coolants can vary widely. In general, at least about 0.5%, by weight, will be added if used for cooling purposes, preferably at least about 1%. Maximum levels are governed by practical concerns and the degree of cooling sensation desired, as well as the desire to minimize possible stinging,

and will generally be no more than about 40%, more generally no more than about 30%.

Skin Renewal Agents

Skin renewal agents can also be used in the present compositions, typically at levels of from 0.05% to about 5%, by weight, preferably from about 0.1% to about 2%. These include salicylic acid, alpha-hydroxy acids, such as glycolic acid, lactic acid, and glutaric acid, and other fruit acids.

Additional Components

The compositions of the present invention can comprise a wide range of additional components. Examples of these classes of additional components include: anti-acne agents, anti-foaming agents, anti-microbial agents, antioxidants, binders, biological additives, buffering agents, chelating agents, colorants, cosmetic astringents, cosmetic biocides, denaturants, drug astringents, external analgesics, film formers, fragrance components, pH adjusters, preservatives, reducing agents, skin bleaching agents, sunscreen agents (e.g., ultraviolet light absorbers); solvents such as monohydric $C_1$-$C_4$ alcohols (typically at levels from 0% to about 40%, preferably from 0% to about 20%, more preferably from 0% to about 5%), especially ethanol; etc.

Other nonlimiting examples of these additional components include the following: vitamins and derivatives thereof (e.g., tocopherol, tocopherol acetate, retinoic acid, retinol, retinoids, and the like); polymers for aiding the film-forming properties and substantively of the composition (such as the copolymer of eicosene and vinyl pyrrolidone, an example of which is available from GAF Chemical Corporation as Ganex® V-220); preservatives for maintaining the anti-microbial integrity of the compositions; other anti-acne medicaments (e.g., resorcinol, sulfur, salicylic acid, erythromycin, and the like); and skin bleaching (or lightening agents including but not limited to hydroquinone, kojic acid, antioxidants, chelators and sequestrants.

PROCESS FOR MAKING

The compositions hereof can be made by mixing the ingredients together according to conventional techniques known in the art. In general, the compositions can be made by combining water and the hydrogel forming polymeric gelling agent, mixing, and adding the other water soluble ingredients. The water insoluble phase can then be added with mixing. Medium or high shear mixing may be used to adjust particle size of the insoluble ingredients to the desired level.

METHOD OF USE

The compositions hereof can be used by topically applying an effective amount for conditioning and moisturizing the skin of a human or other mammal, such as via spreading with the hands or an article, or by spraying. In general, from about 0.05 to about 10 g of product per square centimeter of skin is applied, preferably from about 0.05 g to about 3 g per square centimeter.

EXPERIMENTAL

A) Absorptive Capacity "Tea Bag" Test

Absorptive Capacity can be determined by a gravimetric analytical technique using deionized water as the fluid for which Absorptive Capacity of the polymeric gelling agent is to be calculated. A sample of polymeric gelling agent is placed within a tea bag, immersed in an excess of deionized water for a specified period of time, and then centrifuged for a specific period of time. The ratio of polymeric gelling agent final weight after centrifuging minus initial weight (net fluid gain) to initial weight determines the Absorptive Capacity.

The following procedure is conducted under standard laboratory conditions at 23°C (73°F) and 50% relative humidity. Using a 6 cm X 23 cm cutting die, the tea bag material is cut, folded in half lengthwise and sealed along two sides with a T-bar sealer to produce a 6 cm X 6 cm tea bag square. The tea bag material utilized is a grade 1234 heat sealable material, obtainable from C. H. Dexter, Division of the Dexter Corp., Windsor Locks) Connecticut, U.S.A., or equivalent. Lower porosity tea bag material should be used if required to retain fine particles or fibers of polymeric gelling agent. 0.200 grams plus or minus 0.005 grams of the polymeric gelling agent is weighed onto a weighing paper and transferred into the tea bag and the top (open end) of the tea bag is sealed. An empty tea bag is sealed at the top and is used as a blank. Approximately 300 milliliters of deionized water are poured into a 1,000 milliliter beaker. The blank tea bag is submerged in the deionized water. The tea bag containing the polymeric gelling agent (the sample

tea bag) is held horizontally to distribute the material evenly throughout the tea bag. The tea bag is laid on the surface of the deionized water. The tea bag is allowed to wet, for a period of no more than one minute, and then is fully submerged and soaked for 60 minutes. Approximately 2 minutes after the first sample is submerged, a second set of tea bags, prepared identically to the first set of blank and sample tea bags, is submerged and soaked for 60 minutes in the same manner as the first set. After the prescribed soak time has elapsed, for each set of tea bag samples, the tea bags are promptly removed (using tongs) from the deionized water. The samples are then centrifuged as described below. The centrifuge used is a Delux Dynac II Centrifuge, Fisher Model No. 05-100-26, obtainable from the Fisher Scientific Co. of Pittsburgh, PA, or equivalent. The centrifuge should be equipped with a direct read tachometer and an electric brake. The centrifuge is further equipped with a cylindrical insert basket having an approximately 2.5 inch (6.35 cm) high outer wall with an 8.435 inch (21.425 cm) outer diameter, a 7.935 inch (20.155 cm) inside diameter, and 9 rows each of approximately 106 3/32 inch (0-238 cm) diameter circular holes equally spaced around the circumference of the outer wall, and having a basket floor with six 1/4 inch (0.635 cm) diameter circular drainage holes equally spaced around the circumference of the basket floor at a distance of 1/2 inch (1.27 cm) from the interior surface of the outer wall to the center of the drainage holes, or an equivalent. The basket is mounted in the centrifuge so as to rotate, as well as brake, in unison with the centrifuge. The-sample tea bags are positioned in the centrifuge basket with a folded end of the tea bag in the direction of the centrifuge spin to absorb the initial force. The blank tea bags are placed to either side of the corresponding sample tea bags. The sample tea bag of the second set must be placed opposite the sample tea bag of the first set; and the blank tea bag of the second set opposite the blank tea bag of the first set, to balance the centrifuge. The centrifuge is started and allowed to ramp up quickly to a stable speed of 1,500 rpm, a timer is set for 3 minutes. After 3 minutes, the centrifuge is turned off and the brake is applied. The first sample tea bag and the first blank tea bag are removed and weighed separately. The procedure is repeated for the second sample tea bag and the second blank tea bag.

The Absorptive Capacity (AC) for each of the samples is calculated as follows: AC = (sample tea bag weight after centrifuge minus blank tea bag weight after centrifuge minus polymeric gelling agent weight) / (dry polymeric gelling agent).
The Absorptive Capacity value for use herein is the average absorptive capacity of the two samples.

B) Extractable Polymer Content Determination

Depending upon the type of hydrogel-forming crosslinked polymeric gelling agent involved, two different methods are used herein to calculate extractable polymer content. For carboxylic acid-based polymeric gelling agent a potentiometric procedure is used to determine extractables. For sulfonic acid-based polymeric gelling agent, a gravimetric procedure is employed. It should be noted that both of these procedures may provide results that include in the total amount of extractable material those extractable components in the polymeric gelling agent which are not polymeric. Therefore, if a given polymer sample is known or believed to contain significant amounts of non-polymeric extractable material, such non-polymeric extractable material should be removed from the analyte in conventional fashion before running the extractable polymer content determination hereinafter described.

(1) Carboxylic Acid-Based Polymeric Gelling Agent

Extractable polymer content of carboxylic acid-based polymeric gelling agent is determined by admixing the polymeric gelling agent with deionized water for a period of time sufficient to substantially approach equilibrium with respect to extraction of polymer material from the hydrogel which is formed. The hydrogel/deionized water mixture is allowed to settle and a portion thereof is filtered. An aliquot of this filtrate is then taken, and the free acid groups on the polymer material dissolved in this filtrate are titrated to pH 10 with base. All of the carboxylate groups are then titrated to pH 2.7 with acid. These titration data are then used to calculate the amount of extractable polymer in the polymeric gelling agent sample.

(a) Preparation of the Extractable Polymer-Containing Filtrate Samples

1. 0.40 to 0.41 g of polymeric gelling agent is accurately (to ± 0.1 mg) weighed into a 150 ml disposable beaker. If glass beakers are used they must be acid washed prior to use. (Glassware should be washed three times with dilute HCl [conc. HCl diluted 1:4 with dieionized water], then three times with deionized water. This procedure removes trace of detergents and other contaminants which would otherwise interfere with the titration.)
2. 75 ml of deionized water are added.
3. Samples are slowly stirred for a period of time sufficient to reach equilibrium. Equilibrium is generally reached

within 16 hour periods. If extractable polymer content is to be measured as a function of time, then 1, 6, and 16 hour periods are generally sufficient to define the extractables versus time curve.
4. Samples are allowed to settle for 15 minutes.
5. Using a 3 ml disposable syringe and 0.22 micron filters, enough solution is filtered so that a 20 ml aliquot can be taken.

(b) Titration Conditions

1. If the titrations are to be performed manually, great care must be taken to assure that equilibrium is reached after each addition of titrant.
2. A 20 ml aliquot of the filtrate is transferred to a 50 ml disposable beaker. If glass beakers are being used, they must be acid washed prior to use as noted herein before.
3. The aliquot is titrated to pH 10 with 0. 1N NaOH.
4. The aliquot is then back titrated to pH 2.7 with 0. 1N HCI.
5. Step 3 and 4 are performed on 20 ml of deionized water to obtain titration blinks for both steps of the titration.

(c) Calculations

1. The amount of polymerized acid moieties (e.g., acrylic acid) (in millimoles) in the supernatant aliquot ($M_a$) is given by:

$$M_a = (V_a - V_{ab}) \times N_a \text{ millimoles (mm)}$$

where:

$V_a$= The volume (in ml) of acid required to titrate the aliquot to pH 10.;
$V_b$= The volume (in ml) of acid required to titrate 20 mi of deionized water to pH 10; and
$N_a$= The normality (in meq/ml) of the acid (nominally 0.10 meq/ml)

2. The total amount of polymerized acid moieties (e.g. acrylic acid) plus polymerized neutralized acid moieties (e. g., sodium acrylate) (in mm) in the supernatant aliquot ($M_t$) is given by:

$$M_t=(V_b-V_{bb})xN_b \text{ millimoles}$$

where:

$V_b$= The volume (in ml) of base required to titrate the aliquot from pH 10 down to pH 2.7;
$V_{bb}$= The volume (in ml) of base required to titrate 20 ml of deionized water from pH 10 down to pH 2.7; and
$N_b$ = The normality (in meq/mi) of the base (nominally 0.10 meq/ml).

3. The amount of polymerized neutralized acid moieties (e.g., sodium acrylate) (in mm) in the original supernatant aliquot ($M_b$) is given by:

$$M_b=M_t-M_a$$

4. The total amounts of polymerized acid moieties ($W_a$) and polymerized neutralized acid moieties($W_b$) (e.g., acrylic acid plus sodium acrylate) extracted (in mg) are given by:

$$W_a=M_axE_axD \text{ and } W_b=M_bxE_{bx}D$$

where:

$E_a$ = The equivalent weight of add moiety in polyacid moiety (e.g., acrylic acid in polyacrylic acid =72 meq/mg).
$E_b$= The equivalent weight of neutralized acid moiety in neutralized polyacid moiety (e.g., sodium acrylate in sodium polyacrylate = 94 meq/mg).
D = The dilution factor (75 ml/20 ml= 3.75).

5. The percent extractable polymer in the polymeric gelling agent sample (e) is given by:

$$e=((W_a+ W_b) \times 100) /W$$

where: W=The sample weight in mg.

2. Sulfonic Acid-Containing Polymeric Gelling Agent

Extractable polymer content of sulfonic acid-based polymeric gelling agent is determined by a gravimetric procedure wherein hydrogel samples are swollen overnight in deionized water, and the polymer content in the nitrate is gravimetrically determined. The particular procedure of the gravimetric extractables determination are set forth as follows:

Into a 500 ml Erlenmeyer flask is weighed accurately (to +/- 0.1 mg) about 0.25 grams of dry polymeric gelling agent ($W_p$). 250 ml of deionized water is added and the mixture is stirred slowly for 1 hour. After this hour has passed, stirring is stopped, and the swollen gel is allowed to settle for about 16 hours. The supernatant is filtered using a 3 ml disposable syringe and 0.22 micron filter to obtain at least 40 ml of filtrate. Exactly 40 ml of filtrate is placed into a clean 100 ml round-bottomed flask, and the solution is concentrated on a rotary evaporator (water aspirator vacuum, bath temperature 55°C). The remaining 2-3 ml of solution is transferred quantitatively to a tared weighing vial with the aid of additional deionized water. The solution in the weighing vial is reduced to dryness in an oven at 120°C. The vial is cooled, reweighed, and the weight of residue (Wr) is determined using the tare weight of the vial. The percent extractable polymer (e) is calculated from the weight of dry polymer ($W_p$) and weight of residue ($W_r$) by the following equation:

$$e = (W_r \times 250 \times 100)/(W_p \times 40)$$

EXAMPLES

Below are examples of the present invention which are provided for purposes of exemplifying specific embodiments of the invention, and are not intended to necessarily limit the invention thereto. The scope of the invention is defined in the claims which follow.

| Ingredient (wt. %) | 1 | 2 | 3 | 4 | 5 |
|---|---|---|---|---|---|
| Water | q.s. | q.s. | q.s. | q.s. | q.s. |
| Hydrogel Forming Polymeric Gelling Agent[1] | 0.55 | 0.35 | 0.40 | 0.55 | 0.25 |
| Polymeric Gelling Agent[2] | - | 0.15 | 0.10 | - | 0.25 |
| Silicone Emollient[3a] | 2.00 | 5.00 | 3.00 | 1.00 | 1.50 |
| Silicone Emollient[3b] | - | - | 3.00 | 2.00 | 1.00 |
| Triethanolamine | 0.05 | 0.20 | 0.15 | 0.05 | 0.55 |
| Glycerol | 3.00 | 3.00 | 2.50 | 1.00 | 4.50 |
| PEG-4[4] | 6.00 | - | 5.00 | - | 8.00 |
| PEG-8[5] | - | 12.00 | - | 6.00 | - |

1 Sanwet IM-3000 (Hoechst Celanese/Fine Chemicals)
2 Carbopol 980 (B. F. Goodrich)
3 [a] 87%/13% Cyclomethicone/Dimethiconol (DCQ2-1401 Fluid-Dow Corning)
3 [b] 87%/13% Dimethicone/Dimethiconol (DC22-1403 Fluid-Dow Corning)
4 Polyethylene glycol having an average degree of ethoxylation of 4
5 Polyethylene glycol having an average degree of ethoxylation of 8

(continued)

| Ingredient (wt. %) | 1 | 2 | 3 | 4 | 5 |
|---|---|---|---|---|---|
| Water | q.s. | q.s. | q.s. | q.s. | q.s. |
| PEG-2000[6] | - | - | 0.25 | - | 0.30 |
| PEG-9000[7] | - | 0.10 | - | 0.20 | - |
| Tocopheryl Acetate | 0.25 | 0.50 | - | 0.20 | 0.25 |
| PEG-14,000[8] | 0.40 | - | - | - | - |
| Perfume | 0.75 | 1.00 | 0.50 | 0.75 | 1.50 |
| Sensate[9] | 0.06 | 0.08 | 0.10 | 0.15 | 0.05 |
| Disodium EDTA | 0.02 | 0.02 | 0.02 | 0.02 | 0.02 |
| DMDM Hydantoin (and) Iodopropynyl Butylcarbamate[10] | 0.20 | 0.20 | 0.20 | 0.20 | 0.20 |
| Color (powder) | 0.001 | 0.001 | 0.001 | 0.001 | 0.001 |
| Benzophenone-4 | 0.05 | 0.05 | 0.05 | 0.05 | 0.05 |
| Dexpanthenol | - | - | 0.50 | 0.50 | 1.00 |

6 Polyethylene Glycol having an average degree of ethoxylation of about 2000 (Polyox WSR N-10, Union Carbide).
7 Polyethylene Glycol having an average degree of ethoxylation of about 9000 (Polyox WSR N-3333, Union Carbide).
8 Polyethylene Glycol having an average degree of ethoxylation of about 14,000 (Polyox WSR N-205, Union Carbide).
9 N,2,3-trimethyl-2-isopropyl-butanamide and N-ethyl-p-menthane-3-carboxamide (Sterling Organics, Limited).
10 Glydant Plus[TN], (Lonza, Incorporated).

The above formulations are made as follows. Water is added to a mixing vessel equipped with a stirrer and rotor-stator homogenizer. The water soluble components are added (Benzophenone-4, Triethanolamine, Glydant Plus[TN], Disodium EDTA, Glycerol, Dexpanthenol, PEG-4, PEG-2000 Color) are added with adding. Stirring is continued until the solution is clear. The hydrogel forming polymeric gelling agent (1) is added with mixing. The mixture is then homogenized until a smooth texture is achieved (Premix A). If polymeric gelling agent (2) is used, it is added to water in a separate vessel and stirred until a uniform, dispersed mixture is obtained. The mixture is then added with stirring to Premix A after Premix A has been homogenized. In a separate mixing vessel equipped with a stirrer, the water insoluble materials, other than the silicone emollient, are added (Perfume, Vitamin E Acetate, Sensate). Mixing is continued until all solids are dissolved (Premix B). Premix B is added to Premix A with stirring. Mixing should continue until homogenous. The Silicone Emollient is then added slowly with stirring. Particle size of the dispersed phases may be adjusted to smaller sizes by increasing the mixing speed or use of a blade mixer or homogenizer.

## Claims

**1.** A topical skin care composition comprising:

(a) from 0.1% to 10%, by weight, hydrogel forming polymeric gelling agent, calculated on dry polymeric gelling agent weight basis, said gelling agent not having a Tg below 140°C;
(b) an insoluble silicone emollient dispersed in said composition, said silicone emollient comprising a mixture of (i) from 0.05% to 2%, by weight of the composition, of water insoluble silicone gum; and (ii) from 0.5% to 15%, by weight of the composition, of a water insoluble diluent having a viscosity at 25°C of 300 mPa·s (centipoise) or less;
(c) from 0.5% to 10%, by weight, glycerol, a polymer of glycerol having a weight average molecular weight of 800 or less, alkyl ether derivatives thereof, or a combination thereof;
(d) from 0.5% to 30%, by weight, of polyethylene glycol having a number average degree of ethoxylation of from 2 to 12;
(e) from 0.05% to 2%, by weight, polyoxyethylene having the structure:

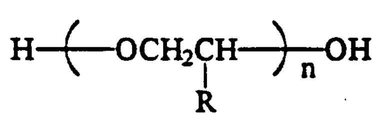

wherein n has a number average of from 800 to 20,000, and R is hydrogen or $C_1$ to $C_4$ alkyl, or a combination thereof;
(f) from 50% to 98.7%, by weight, water.

**2.** A skin care composition as in Claim 1, wherein R of said polyoxyethylene is hydrogen.

**3.** A skin care composition as in Claim 1, wherein said water insoluble diluent is a silicone fluid, a hydrocarbon fluid, or a mixture thereof.

**4.** A skin care composition as in Claim 1, wherein said hydrogel forming polymeric gelling agent is **characterized by** not having a Tg below 180°C.

**5.** A skin care composition as in Claim 1, wherein said water insoluble diluent has a viscosity of less 100 mPa·s (centipoise) at 25°C.

**6.** A skin care composition as in Claim 5, wherein said diluent is silicone and has a viscosity of from 0.65 centipoise to 50 mPa·s (centipoise) at 25°C.

**7.** A skin care composition as in Claims 1, 3 or 6, wherein said silicone gum is dimethicone, dimethiconol, or a mixture thereof.

**8.** A skin care composition as in Claim 1, wherein said composition further comprises:

(g) a carboxylic acid containing polymer **characterized by** having a Tg at a temperature of below 140°C; wherein the weight ratio of (a):(g) is from 100:0 to 50:50, preferably from 90:10 to 60:40.

**9.** A skin care composition as in Claim 1, wherein the mole weight of (c):(d) is from 2:3 to 3:2.

**10.** A non-therapeutic method for treating the skin comprising applying an effective amount of the composition of Claim 1 for conditioning and moisturizing said skin.

**Patentansprüche**

**1.** Topische Hautpflegezusammensetzung, umfassend

(a) 0,1 Gew.-% bis 10 Gew.-% eines ein Hydrogel ausbildenden polymeren Geliermittels, berechnet auf Trokkengewichtsbasis des polymeren Geliermittels, welches Geliermittel keine Tg unter 140°C besitzt;
(b) ein unlösliches, in der genannten Zusammensetzung dispergiertes Silikonemolliens, welches Silikonemolliens ein Gemisch aus (i) 0,05 Gew.-% bis 2 Gew.-% der Zusammensetzung von wasserunlöslichem Silikonkautschuk und (ii) 0,5 Gew.-% bis 15 Gew.-% der Zusammensetzung von einem wasserunlöslichen Verdünnungsmittel mit einer Viskosität bei 25°C von 300 mPas (Centipoise) oder weniger umfaßt;
(c) 0,5 Gew.-% bis 10 Gew.-% Glycerin, von einem Glycerinpolymer mit einem Gewichtsmittel-Molekulargewicht von 800 oder weniger, Alkyletherderivaten hievon, oder einer Kombination hievon;
(d) 0,5 Gew.-% bis 30 Gew.-% Polyethylenglycol mit einem zahlenmittleren Ethoxylierungsgrad von 2 bis 12;
(e) 0,05 Gew.-% bis 2 Gew.-% Polyoxyethylen der Struktur

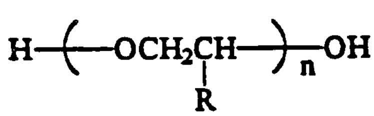

worin n ein Zahlenmittel von 800 bis 20.000 besitzt und R Wasserstoff oder $C_1$-$C_4$-Alkyl oder eine Kombination hievon ist;
(f) 50 Gew.-% bis 98,7 Gew.-% Wasser.

**2.** Hautpflegezusammensetzung nach Anspruch 1, worin R im genannten Polyoxyethylen Wasserstoff darstellt.

**3.** Hautpflegezusammensetzung nach Anspruch 1, worin das genannte wasserunlösliche Verdünnungsmittel, ein Silikonöl, ein Kohlenwasserstofföl oder ein Gemisch hievon ist.

**4.** Hautpflegezusammensetzung nach Anspruch 1, worin das genannte das Hydrogel ausbildende polymere Geliermittel **dadurch gekennzeichnet ist, daß** es keine Tg unter 180°C besitzt.

**5.** Hautpflegezusammensetzung nach Anspruch 1, worin das genannte wasserunlösliche Verdünnungsmittel eine Viskosität bei 25°C von weniger als 100 mPas (Centipoise) aufweist.

**6.** Hautpflegezusammensetzung nach Anspruch 5, worin das genannte Verdünnungsmittel Silikon ist und eine Viskosität bei 25°C von 0,65 bis 50 mPas (Centipoise) aufweist.

**7.** Hautpflegezusammensetzung nach den Ansprüchen 1, 3 oder 6, worin der genannte Silikonkautschuk Dimethicon, Dimethiconol oder ein Gemisch hievon ist.

**8.** Hautpflegezusammensetzung nach Anspruch 1, worin die genannte Zusammensetzung ferner

(g) ein Carbonsäure enthaltendes Polymer umfaßt, welches **dadurch gekennzeichnet ist, daß** es eine Tg bei einer Temperatur unter 140°C besitzt; wobei das Gewichtsverhältnis von (a):(g) von 100:0 bis 50:50, vorzugsweise von 90:10 bis 60:40 beträgt.

**9.** Hautpflegezusammensetzung nach Anspruch 1, worin das Molgewichtsverhältnis von (c):(d) von 2:3 bis 3:2 beträgt.

**10.** Nichttherapeutisches Verfahren zur Behandlung der Haut, umfassend das Aufbringen einer wirksamen Menge der Zusammensetzung nach Anspruch 1 zur Konditionierung und Befeuchtung der Haut.

## Revendications

**1.** Composition topique pour les soins de la peau, comprenant :

(a) de 0,1 à 10 % en poids d'un agent de gélification polymère formant un hydrogel, calculés sur la base du poids sec de l'agent de gélification polymère, ledit agent de gélification n'ayant pas une $T_v$ inférieure à 140°C ;
(b) un émollient constitué d'une silicone insoluble, en dispersion dans ladite composition, ledit émollient constitué d'une silicone comprenant un mélange (i) de 0,05 à 2 % en poids, par rapport à la composition, d'une gomme de silicone insoluble dans l'eau ; et (ii) de 0,5 à 15 % en poids, par rapport à la composition, d'un diluant insoluble dans l'eau ayant une viscosité à 25°C de 300 mPa.s (centipoises) ou moins ;
(c) de 0,5 à 10 % en poids de glycérol, d'un polymère du glycérol ayant une masse moléculaire moyenne en masse de 800 ou moins, de leurs dérivés éthers alkyliques, ou d'une de leurs combinaisons ;
(d) de 0,5 à 30 % en poids d'un polyéthylèneglycol ayant un degré moyen d'éthoxylation en nombre de 2 à 12 ;
(e) de 0,05 à 2 % en poids d'un polyoxyéthylène ayant la structure

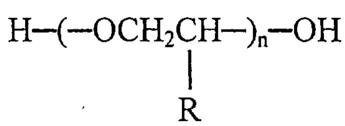

dans laquelle n a une valeur moyenne en nombre de 800 à 20 000, et R est un atome d'hydrogène ou un groupe alkyle en $C_1$ à $C_4$, ou une de leurs combinaisons ;
(f). de 50 à 98,7 % en poids d'eau.

**2.** Composition pour les soins de la peau selon la revendication 1, dans laquelle R, dans ledit polyoxyéthylène, est un atome d'hydrogène.

**3.** Composition pour les soins de la peau selon la revendication 1, dans laquelle ledit diluant insoluble dans l'eau est un fluide de silicone, un fluide hydrocarboné ou un de leurs mélanges.

**4.** Composition pour les soins de la peau selon la revendication 1, dans laquelle ledit agent de gélification polymère formant un hydrogel est **caractérisé en ce qu'**il n'a pas une $T_v$ inférieure à 180°C.

**5.** Composition pour les soins de la peau selon la revendication 1, dans laquelle ledit diluant insoluble dans l'eau a une viscosité inférieure à 100 mPa.s (centipoises) à 25°C.

**6.** Composition pour les soins de la peau selon la revendication 5, dans laquelle ledit diluant est une silicone et a une viscosité de 0,65 à 50 mPa.s (centipoises) à 25°C.

**7.** Composition pour les soins de la peau selon les revendications 1, 3 ou 6, dans laquelle ladite gomme de silicone est la diméthicone, le diméthiconol ou un de leurs mélanges.

**8.** Composition pour les soins de la peau selon la revendication 1, dans laquelle ladite composition comprend en outre

(g) un polymère contenant un acide carboxylique, **caractérisé en ce qu'**il a une $T_v$ à une température inférieure à 140°C ; où le rapport en poids (a):(g) est de 100:0 à 50:0 et de préférence de 90:10 à 60:40.

**9.** Composition pour les soins de la peau selon la revendication 1, dans laquelle le rapport en moles (c):(d) est de 2:3 à 3:2.

**10.** Procédé non thérapeutique de traitement de la peau, qui comprend l'application d'une quantité efficace de la composition selon la revendication 1 pour conditionner et humidifier ladite peau.